# EUROPEAN PATENT SPECIFICATION

(11) **EP 0 633 879 B1**
(45) Date of publication and mention of the grant of the patent: **11.03.1998**
(21) Application number: 93907975.2
(22) Date of filing: 01.04.1993
(51) Int. Cl.: C07D 213/38, A61K 31/44

(54) **ENANTIOMERIC 1-PHENYL-2-(2-PYRIDINYL)ETHYLAMINE FOR THE TREATMENT OF NEURODEGENERATIVE DISORDERS**
ENANTIOMERE 1-PHENYL-2-(2-PYRIDINYL)ETHYLAMIN FÜR DIE BEHANDLUNG NEURODEGENERATIVER STÖRUNGEN
1-PHENYL-2-(2-PYRIDINYL)ETHYLAMINE ENANTIOMERE POUR LE TRAITEMENT DE MALADIES DE DEGENERESCENCE DU SYSTEME NERVEUX

(30) Priority: 03.04.1992 GB 9207339; 15.04.1992 GB 9208290
(43) Date of publication of application: 18.01.1995
(73) Proprietor: Astra Aktiebolag, 151 85 Södertälje (SE)
(72) Inventor: GRIFFITH, Ronald, Conrad, Pittsford, NY 14534 (US); MURRAY, Robert, John, Brighton, NY 14618 (US); BALESTRA, Michael, Rochester, NY 14624 (US)
(74) Representative: Summersell, Richard John, Dr.
(86) International application number: GB9300689
(87) International publication number: WO9320052

(56) References cited:
- EP-A- 0 356 035
- J.B. TAYLOR 'Comprehensive medicinal chemistry Vol.5 Biopharmaceutics' , PERGAMON PRESS , OXFORD

## Description

This invention relates to an enantiomer of a known compound, its use as a pharmaceutical, in particular in the treatment of neurodegenerative disorders, a process for its production, and pharmaceutical formulations containing it.

A major problem with existing drugs used to treat neurodegenerative disorders is a lack of predictability in the concentration of a drug in a patient's blood plasma resulting from administration of a given quantity of that drug, i.e. existing drugs do not exhibit linear pharmacokinetics. It has been stated that an ideal drug in this field would show a linear relationship between blood plasma concentration and dose size so that a given change in dose would yield a predictable change in blood plasma concentration of the drug ['Pharmacokinetics of old, new and yet-to-be discovered antiepileptic drugs', R H Levy and B M Kerr, Epilepsia, vol 30, Supp 1, S35-S41, 1989].

European Patent Application 356035 discloses a large number of compounds for use in the treatment of neurodegenerative disorders, including α-phenyl-2-pyridineethanamine [referred to therein as 1-phenyl-2-(2-pyridinyl)ethylamine],

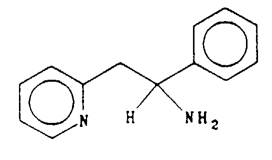

Surprisingly, it has now been found that the (S)-enantiomer of this compound exhibits linear pharmacokinetics, whereas the racemate exhibits nonlinear pharmacokinetics.

Thus, according to the present invention, there is provided (S)-α-phenyl-2-pyridineethanamine, 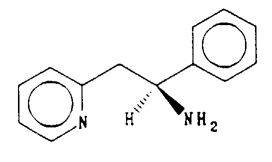 substantially free from its (*R*)-enantiomer, and pharmaceutically acceptable derivatives thereof (hereinafter referred to together as "the compounds of the invention").

By "substantially free from its (*R*)-enantiomer", is meant that a sample of the (*S*)-enantiomer contains less than 10% by weight of the (*R*)-enantiomer (i.e. it is more than 90% enantiopure), more preferably less than 1% by weight of the (*R*)-enantiomer, and most preferably is pure (*S*)-enantiomer.

Pharmaceutically acceptable derivatives include compounds which are suitable bioprecursors (prodrugs) of (*S*)-α-phenyl-2-pyridineethanamine, and of particular interest - acid addition salts.

Suitable bioprecursors of (+)-α-phenyl-2-pyridineethanamine include amino acid amide derivatives of the amino group, in particular α-amino acid derivatives such as glycine derivatives. Such derivatives may be prepared by conventional methods, for example amino acid amide derivatives may be prepared by the methods given in 'Advanced Organic Chemistry' by J March, 2nd edition, published by McGraw-Hill, page 1171.

Acid addition salts of (*S*)-α-phenyl-2-pyridineethanamine include salts of mineral acids, for example the dihydrochloride and dihydrobromide salts; and salts formed with organic acids such as formate, acetate. malate, benzoate and fumarate salts.

α-Phenyl-2-pyridineethanamine may be prepared by conventional methods (for example, addition of the anion of 2-picoline to N-trimethylsilyl-benzaldimine). (*S*)-α-phenyl-2-pyridineethanamine may then be prepared by one or more selective precipitations of a diastereomeric salt formed by reaction of α-phenyl-2-pyridineethanamine with a chiral salt, followed by one or more recrystallizations. Thus, according to a second aspect of the invention, there is provided a process for the preparation of a compound of the invention which comprises selective precipitation of a diastereomeric salt formed between α-phenyl-2-pyridineethanamine and a chiral acid. Chiral acids which may be mentioned include D- or L-tartaric acids and in particular S(+)- and R(-)-mandelic acids. The precipitation may be carried out in an organic solvent which does not adversely affect the reaction (for example ethyl acetate), at or around room temperature.

The compounds of the invention are indicated as pharmaceuticals, in particular as anticonvulsants and neuroprotective agents in the treatment of neurodegenerative disorders. Specific neurodegenerative disorders that may be mentioned include stroke, cerebral ischaemia, cerebral palsy, the effects of hypoglycaemia, epilepsy, AIDS-related dementia, Alzheimer's disease, Huntington's chorea, Olivo-ponto-cerebellar atrophy, perinatal asphyxia, Parkinson's disease, anoxia, neuronal damage associated with substance abuse (for example, narcotics or cocaine), retinopathies, schizophrenia, ischaemic states after cardiac arrest or surgical operations, intoxication or injuries of the spinal cord and amyotrophic lateral sclerosis.

While not being limited by theory, neurodegeneration is thought to be caused or accelerated by certain excitatory amino acids found naturally in the central nervous system (CNS). Glutamate is an endogenous amino acid which has been characterized as a fast excitatory transmitter in the mammalian brain. Glutamate is also known as a powerful neurotoxin capable of killing CNS neurons under certain pathologic conditions which accompany stroke and cardiac arrest. It has been shown that the sensitivity of central neurons to hypoxia and ischaemia can be reduced by the specific antagonism of post synaptic glutamate receptors. Glutamate is characterized as a broad spectrum agonist having activity at four neuronal excitatory amino acid receptor sites. These receptor sites are named after the amino acids which selectively excite them: kainate (KA), N-methyl-D-aspartate (NMDA), quisqualate (QUIS) and 2-amino-4-phosphonobutyrate (APB). Glutamate is believed to be a mixed agonist capable of binding to and exciting all four receptor types. Thus, agents which selectively block or antagonise the action of glutamate at these receptors can prevent neurotoxic injury associated with anoxia, hypoxia or ischemia. In particular, compounds which bind to the NMDA receptor site and selectively block the action of glutamate are useful in the prevention and treatment of neurodegenerative diseases.

The pharmacological activity of the compounds of the invention may be measured in the tests set out below.
a) **NMDA blocking activity** is measured by assessing a compound's ability to protect mice from convulsions induced by intravenous administration of 150mg/kg of NMDA according to the procedures of Czuczwar et al, (Neurotransmitters. Seizures and Epilepsy III, edited by G Nistico et al. Raven Press. New York 1986, pages 235-246). Groups of mice are pretreated by 30 minutes with the test compound by the intraperitoneal routes and then given NMDA. Animals were observed for convulsions as defined by loss of righting reflex and appearance of tonic/clonic seizures. Animals are kept for 60 minutes after NMDA dosing and mortality was recorded.
b) **NMDA receptor antagonist activity** may be measured in vitro by assaying a compound's ability to inhibit binding of the receptor antagonist 10,11-dihydro-5-methyl-5H-dibenzo[a,d]-cyclohepten-5,10-imine (MK 801) to the receptor. The method is described by Foster and Wong, Br J Pharmacol 91, 403-409 (1987).
c) **NMDA and glycine receptor affinity** may also be tested in the [³H]L-glutamate and [³H]glycine binding assays following the method of Monaghan & Cotman, PNAS, 83, 7532, (1986) and Watson et al, Neurosci Res Comm, 2, 169, (1988).
d) **Antihypoxia activity** may be measured conveniently in mice. Groups of mice are tested at various times after the intraperitoneal administration of graded doses of the test compound. The animals' survival time in a temperature-controlled hypoxic environment (96% nitrogen and 4% oxygen) is recorded. A statistical comparison is made between coincident vehicle treated animals and the experimental group. The dose-response and minimum active dose (MAD) for compounds are obtained [A A Artu and J D Michenfelder, Anaesthesia and Analgesia, 1981, 60, 867]. Other modes of administration can also be used.
e) **Antiepileptic activity** may be measured by assessing a compound's ability to prevent the hind limb tonic extension component of the seizure in groups of mice or rats induced by maximal electroshock (MES) after oral, intraperitoneal. intravenous or subcutaneous administration, according to the procedures of the Epilepsy Branch, NINCDS as published by R J Porter, et al, Cleve Clin Quarterly 1984, 51, 293, and compared with the standard agents dilantin and phenobarbital.
f) **The 4-vessel occlusion (4-VO) model of stroke** is used to produce global ischaemia in the rat and is an essential technique to evaluate the effectiveness of compounds to prevent damage to areas of selective vulnerability in the brain, notably the CA1 pyramidal neurons of the hippocampus. This area is involved in the pathways for short term memory formation in both laboratory animals and humans. The procedure consists of cauterizing the vertebral arteries and isolating the carotid arteries of rats maintained under anaesthesia on day 1. On day 2 the carotids are clamped for varying periods of time, ten minutes is sufficient to destroy the CA1 neurons. The clamps are removed, reflow initiated and drugs administered at various times post reflow. Body temperature is maintained at 37°C throughout the ischaemia and recovery periods. The CA1 neurons die off over a 48-72 hour period and normally the rats are treated for at least 3 days with drug (ip, iv, or po) and at 7 days the brains are removed for histology. Rating of CA1 damage is accomplished using two methods, counting of viable CA1 neurons and scoring of degree of gross pathology [W A Pulsinelli and A Buchan, 'The NMDA receptor/ion channel: Its importance to in vivo ischemia injury to selectively vulnerable neurons', Pharmacology of Cerebral Ischemia, edited by J Krieglstein and H Oberpichler, published by Wissenschaftliche Verlagsgesellschaft, Stuttgart, 1990, p169].
g) In the **Focal Model of Stroke,** spontaneously hypertensive rats (SHR) are used as experimental subjects because of their relatively poor collateral brain circulation. A 2 hour focal ischemia is achieved in SHR by clamping the middle cerebral artery and the ipsilateral carotid while maintaining anaesthesia. Drugs can be administered (usually
ip) either before or various times after clamping the arteries or when reflow commences at 2 hours. The brains are removed 24 hours after the experiment and frozen, sectioned and drug effects toward reducing infarct volume of the cerebral cortex is determined using a custom-built computer quantification system [A M Buchan, D Xue and A Slivka, Stroke, 1992, 23, 273.]

Toxicity of the compounds of the invention may be measured in the following tests.
a) **Dose ranging studies** based on those described by N W Spurling and P F Carey, 'A protocol for dose selection in repeat dose toxicity studies', poster presentation 974 at the Society of Toxicology annual meeting, Seattle, USA, 23-27 February 1992. Rats are dosed intravenously daily with progressively increasing doses of test compound until a maximum repeatable dose is found above which the incidence of convulsions and other abnormal clinical signs is unacceptable.
b) **The inverted screen test** [L L Cougenour. J R McLean, and R B Parker. Pharmacol Biochem Behav, 1977, 6, 351]. Mice are dosed with test compound and 30 minutes later are placed on a small wire platform which is inverted through an arc of 180°. Mice unable to climb to the upright position within 30 seconds are rated as failures. Using sufficient doses and numbers of animals an appropriate TD₅₀ (dose in which 50% fail) can readily be determined.
c) **The observation test for 28 behavioral signs** according to S Irwin [Psychopharmacology 1968, 13, 222]. Groups of 3 mice per dose are administered incremental amounts of test compound in the range 25-400 mg/kg and observed for 28 symptoms immediately after dosing, 30 minutes, 3 and 24 hours post dose.
d) **Test for Phencyclidine (PCP)-Like Behaviour.** PCP-like behaviours are a side effect of potent competitive and non-competitive NMDA receptor antagonists. In a screen to determine whether a compound possesses this liability, rats are dosed orally with test compound (expressed as multiples of the oral ED₅₀ for protection in the MES test) and placed into individual clear plastic cages and observed over a 4 hour period for any incidence of 5 characteristic behaviours associated with PCP, namely hyperactivity, ataxia, circling, head weaving and retropulsion. Five rats per treatment group are observed and compared to a control group receiving PCP. A total incidence score would be 25, i.e. 5 rats exhibiting all 5 behaviours. PCP at 10 times the ED₅₀ produces a score of 25 [W Koek, J H Woods, P Ornstein, 1987, Psychopharmacology, 91, 297].
e) **Gang Plank Escape Test** to measure neural impairment in rats [G E Garske *et al*, Epilepsy Research, 1991, 9, 161]. Rats are placed on a narrow board (1.25cm wide suspended 40cm above the bench top) in a well lit entry cubicle which enters a progressively darkened box connected to a dark escape cubicle at the other end (board is 63cm long). A rat is impaired if it fails to negotiate the plank. The task takes into account two known behaviours of rats, i.e. fear of height and seeking a dark environment.

**Linear pharmacokinetics** may be detected in rats by evaluating the area under the plasma concentration ν time curves obtained upon single intravenous administration of test compound at increasing doses (Smith *et al,* Xenobiotica, 20, 1187-1199. 1990).

Blood was removed from a jugular vein catheter at various times over a 24 hour period. The plasma was separated by centrifugation and the concentration of test compound was determined using HPLC-UV chromatography. The plasma concentration ν time values were plotted for each dose and the area under each curve estimated. Where linear pharmacokinetics are present, the area under the plasma concentration ν time curve for a given dose is directly proportional to the dose administered. A finding of linear pharmacokinetics in rats indicates that linear pharmacokinetics would be found in humans (Leander *et al,* Epilepsia, 33, 696-704, 1992, at p703).

According to another aspect of this invention there is provided a method of treatment of a neurodegenerative disorder, which comprises administering a therapeutically effective amount of a compound of the invention to a patient. Of particular interest is such a method in which the dose of the compound administered is linearly proportional to the blood plasma concentration of the compound desired.

For the above-mentioned uses the dosage administered will, of course, vary with the compound employed, the mode of administration and the treatment desired. However, in general, satisfactory results are obtained when the compounds of the invention are administered at a daily dosage of from about 0.lmg to about 20mg per kg of animal body weight, preferably given in divided doses 1 to 4 times a day or in sustained release form. For man, the total daily dose is in the range of from 5mg to 1,400mg, more preferably from 10mg to 100mg, and unit dosage forms suitable for oral administration comprise from 2mg to 1,400mg of the compound admixed with a solid or liquid pharmaceutical carrier or diluent.

The compounds of the invention may be used on their own or in the form of appropriate medicinal preparations for enteral or parenteral administration. According to a further aspect of the invention, there is provided a pharmaceutical composition comprising preferably less than 80% and more preferably less than 50% by weight of a compound of the invention in admixture with a pharmaceutically acceptable adjuvant, diluent or carrier.

Examples of diluents and carriers are:
for tablets and dragees: lactose, starch, talc, stearic acid:
for capsules: tartaric acid or lactose;
for injectable solutions: water, alcohols, glycerin, vegetable oils;
for suppositories: natural or hardened oils or waxes.

An adjuvant of particular interest when the compound of the invention is to be used in the treatment of Parkinson's disease is L-dopa.

According to a further aspect of the invention, there is provided the use of a compound of the invention as active ingredient in the manufacture of a medicament for the treatment of a neurodegenerative disorder.

The compounds of the invention may also have the advantage that they are less toxic, more efficacious, are longer acting, have a broader range of activity, are more potent, produce fewer side effects, are more easily absorbed or have other useful pharmacological properties than compounds previously indicated in the therapeutic fields mentioned above.

The invention is illustrated by the following examples.

### Example 1

### Preparation of (S)-α-phenyl-2-pyridineethanamine dihydrochloride

### a) α-Phenyl-2-pyridineethanamine dihydrochloride

To a cooled (0°C) solution of benzaldehyde (34.24g, 0.323 moles) in 600ml of tetrahydrofuran (THF) was added lithium bis(trimethylsilyl)-amide (LHMDS) (323ml of a 1.0M solution in THF, 0.323 moles) dropwise over 30 minutes. This mixture was stirred at 0°C for three hours.

In a separate round bottom flask containing a cooled (-78°C) solution of 2-picoline (30.0g, 0.323 moles) in THF (600ml) was added n-butyllithium (n-BuLi) (129.2ml of a 2.5M solution in hexane) over twenty minutes.

The first reaction mixture was allowed to warm to 0°C and remain there for an additional forty minutes. The second reaction mixture (containing the lithiated anion of 2-picoline) was cannulated into the first reaction mixture over 20 minutes. After 30 additional minutes the cold bath was removed and the mixture was allowed to warm to ambient temperature. After an additional one hour, the reaction mixture was poured into a separating funnel charged with ice (11) and 12 N HCl (200ml). The aqueous layer was washed with 3x200ml of diethyl ether (Et₂O) and then basified with 25% NaOH solution in water. The aqueous layer was extracted with 2x200ml of chloroform, the chloroform extracts dried over MgSO₄, filtered and concentrated in vacuo. The residue was dissolved in ethyl acetate (EtOAc) and acidified with a saturated solution of HCl/EtOAc. The solution was diluted with Et₂O and the resulting white solid filtered and dried in vacuo to give the subtitle compound (37.08g, 43%), mp = 206-208°C.

### b) (S)-α-Phenyl-2-pyridineethanamine dihydrochloride

To a solution of racemic α-phenyl-2-pyridineethanamine (the free base of the product of step (a), obtained by neutralizing an aqueous solution of the product of step (a) with a 25% NaOH solution in water and extracting with chloroform) (10.96g, 0.0553 moles) in EtOAc (400ml) was added a solution of S(+)-mandelic acid (8.41g, 0.0553 moles) in EtOAc (300ml). The resulting precipitate was recrystallized from hot EtOAc (500ml) an additional three times. The salt was basified with a 25% NaOH solution in water, extracted With 3x100ml of chloroform, dried over MgSO₄, filtered and concentrated in vacuo. The residue was dissolved in EtOAc (300ml) and acidified with a saturated solution of HCl/EtOAc. The resulting white solid is filtered and dried in vacuo to give (-)-α-phenyl-2-pyridineethanamine dihydrochloride (5.5g), mp = 220-222°C, [α]_{D} = -87.3° (c = 1.0, CH₃OH).

The filtrate from the initial precipitation was neutralized with 25% NaOH solution in water, extracted with 2x250ml of CHCl₃, dried over MgSO₄, filtered and concentrated in vacuo. The residue was dissolved in EtOAc (500ml) and to this solution was added a solution of R(-)-mandelic acid (6.5g, 0.043 moles) in EtOAc (500ml). The precipitate was filtered off and recrystallized an additional three times. The salt was basified with 25% NaOH solution in water, extracted with 3x100ml of chloroform. dried over MgSO₄, filtered and concentrated in vacuo. The residue was dissolved in EtOAc (300ml) and acidified with a saturated solution of HCl/EtOAc. The resulting white solid was filtered and dried in vacuo to give the title compound (3.84g), mp = 220-222°C, [α]_{D} = +87.1° (c = 1.1, CH₃OH).

The enantiopurity may be determined by derivatizing either the mandelic acid or dihydrochloride salt with enantiopure (greater than 99.5%) methylbenzyl isocyanate, and then analyzing by HPLC using a normal phase column with ethanol/hexane [6:94] as solvent. The enantiopurity of the enantiomers obtained above was shown to be greater than 99.5%.

X-ray crystallography showed the (+)-enantiomer to have (S)-absolute stereochemistry.

### Example 2

The compound of Example 1 was found to have an activity (ED₅₀) of 3.7mg/kg in the prevention of hind limb tonic extension in rats induced by maximal electroshock (MES) (described above) when administered orally. Its enantiomer had an ED₅₀ of 20.2mg/kg.

## Claims

1. (*S*)-α-phenyl-2-pyridineethanamine, which is greater than 90% enantiopure, and pharmaceutically acceptable derivatives thereof.

2. (*S*)-α-phenyl-2-pyridineethanamine, which is greater than 99% enantiopure, and pharmaceutically acceptable derivatives thereof.

3. **Pure** (*S*)-α-phenyl-2-pyridineethanamine and pharmaceutically acceptable derivatives thereof.

4. A pharmaceutical formulation **adapted for administration to humans to provide a substantially linear pharmacokinetic response** comprising (*S*)-α-phenyl-2-pyridineethanamine as defined in any one of claims 1 to 3 or a pharmaceutically acceptable derivative thereof, in admixture with a pharmaceutically acceptable adjuvant, diluent or carrier.

5. (*S*)-α-phenyl-2-pyridineethanamine as defined in any one of claims 1 to 3 or a pharmaceutically acceptable derivative thereof, for use as a pharmaceutical.

6. The use of (*S*)-α-phenyl-2-pyridineethanamine as defined in any one of claims 1 to 3, or a pharmaceutically acceptable derivative thereof, as an active ingredient in the manufacture of a medicament for the treatment of a neurodegenerative disorder.

7. A process for the preparation of (*S*)-α-phenyl-2-pyridineethanamine as defined in any one of claims 1 to 3 or a pharmaceutically acceptable derivative thereof, which comprises selective precipitation of a diastereomeric salt formed between α-phenyl-2-pyridineethanamine and a chiral acid.

8. (*S*)-α-phenyl-2-pyridineethanamine as defined in any one of claims 1 to 3 in the form of an acid addition salt.

9. (*S*)-α-phenyl-2-pyridineethanamine as defined in any one of claims 1 to 3 in the form of the dihydrochloride, dihydrobromide, formate, acetate, malate, benzoate or fumarate salt.

## Patentansprüche

1. (*S*)-α-Phenyl-2-pyridinethanamin, das über 90% enantiomerenrein ist, und dessen pharmazeutisch unbedenkliche Derivate.

2. (*S*)-α-Phenyl-2-pyridinethanamin, das über 99% enantiomerenrein ist, und dessen pharmazeutisch unbedenkliche Derivate.

3. (*S*)-α-Phenyl-2-pyridinethanamin und dessen pharmazeutisch unbedenkliche Derivate.

4. Pharmazeutische Formulierung, enthaltend (*S*)-α-Phenyl-2-pyridinethanamin nach einem der Ansprüche 1 bis 3 oder eines seiner pharmazeutisch unbedenklichen Derivate zusammen mit einem pharmazeutisch unbedenklichen Hilfsstoff, Verdünnungsmittel oder Träger.

5. (*S*)-α-Phenyl-2-pyridinethanamin nach einem der Ansprüche 1 bis 3 oder ein pharmazeutisch unbedenkliches Derivat davon zur Verwendung als Pharmazeutikum.

6. Verwendung von (*S*)-α-Phenyl-2-pyridinethanamin nach einem der Ansprüche 1 bis 3 oder einem pharmazeutisch unbedenklichen Derivat davon als Wirkstoff bei der Herstellung eines Arzneimittels zur Behandlung einer neurodegenerativen Erkrankung.

7. Verfahren zur Herstellung von (*S*)-α-Phenyl-2-pyridinethanamin nach einem der Ansprüche 1 bis 3 oder eines seiner pharmazeutisch unbedenklichen Derivate, bei dem man ein diastereomeres Salz aus α-Phenyl-2-pyridinethanamin und einer chiralen Säure selektiv ausfällt.

8. (*S*)-α-Phenyl-2-pyridinethanamin nach einem der Ansprüche 1 bis 3 in Form eines Säureadditionssalzes.

9. (*S*)-α-Phenyl-2-pyridinethanamin nach einem der Ansprüche 1 bis 3 in Form eines Dihydrochlorid-, Dihydrobromid-, Formiat-, Acetat-, Malat-, Benzoat- oder Fumaratsalzes.

## Revendications

1. (*S*)-α-Phényl-2-pyridineéthanamine, qui est pure du point de vue énantiomérique à plus de 90%, et dérivés pharmaceutiquement acceptables de celle-ci.

2. (*S*)-α-Phényl-2-pyridineéthanamine, qui est pure du point de vue énantiomérique à plus de 99%, et dérivés pharmaceutiquement acceptables de celle-ci.

3. (*S*)-α-Phényl-2-pyridineéthanamine et dérivés pharmaceutiquement acceptables de celle-ci.

4. Formulation pharmaceutique comprenant la (*S*)-α-phényl-2-pyridineéthanamine telle que définie dans l'une quelconque des revendications 1 à 3, ou un dérivé pharmaceutiquement acceptable de celle-ci, en mélange avec un adjuvant, diluant ou support pharmaceutiquement acceptable.

5. (*S*)-α-Phényl-2-pyridineéthanamine telle que définie dans l'une quelconque des revendications 1 à 3, ou un dérivé pharmaceutiquement acceptable de celle-ci, à utiliser comme produit pharmaceutique.

6. Utilisation de la (*S*)-α-phényl-2-pyridineéthanamine telle que définie dans l'une quelconque des revendications 1 à 3, ou un dérivé pharmaceutiquement acceptable de celle-ci, comme ingrédient actif dans la préparation d'un médicament pour le traitement d'un désordre neurodégénératif.

7. Procédé de préparation de la (*S*)-α-phényl-2-pyridineéthanamine telle que définie dans l'une quelconque des revendications 1 à 3, ou un dérivé pharmaceutiquement acceptable de celle-ci, qui comprend la précipitation sélective d'un sel diastéréoisomérique formé entre l'α-phényl-2-pyridineéthanamine et un acide chiral.

8. (S)-α-Phényl-2-pyridineéthanamine telle que définie dans l'une quelconque des revendications 1 à 3, sous la forme d'un sel d'addition d'acide.

9. (S)-α-Phényl-2-pyridineéthanamine telle que définie dans l'une quelconque des revendications 1 à 3, sous la forme du sel dichlorhydrate, dibromhydrate, formiate, acétate, malate, benzoate ou fumarate.
